(19) Europäisches Patentamt
European Patent Office
Office européen des brevets

(11) **EP 2 465 561 B1**

(12) **EUROPEAN PATENT SPECIFICATION**

(45) Date of publication and mention
of the grant of the patent:
**19.10.2016  Bulletin 2016/42**

(51) Int Cl.:
***A61M 16/00*** *(2006.01)*

(21) Application number: **11170468.0**

(22) Date of filing: **19.06.2011**

(54) **Ventilator machine with automatic control**

Ventilatormaschine mit automatischer Steuerung

Machine à ventilateur avec commande automatique

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.12.2010  DE 102010055242**

(43) Date of publication of application:
**20.06.2012  Bulletin 2012/25**

(73) Proprietor: **Drägerwerk AG & Co. KGaA
23558 Lübeck (DE)**

(72) Inventors:
• **Eger, Marcus
23562 Lubeck (DE)**
• **Hansmann, Hans-Ullrich
23858 Barnitz (DE)**
• **Glaw, Tobias
23558 Lubeck (DE)**
• **Sattler, Frank
23560 Lubeck (DE)**

(74) Representative: **McCartney, Jonathan William et al
Haseltine Lake LLP
Redcliff Quay
120 Redcliff Street
Bristol BS1 6HU (GB)**

(56) References cited:
**DE-B3-102007 062 214     US-A1- 2010 252 038
US-B1- 6 588 423**

## Description

The present invention relates to a ventilator machine having a source of gas for breathing, a ventilator unit arranged to feed gas for breathing through connecting lines, a control unit arranged to control the ventilator unit, sensors arranged to pick up at least one pneumatic signal for breathing activity which are connected to the control unit; the control unit being arranged to control the ventilator unit of the ventilator machine to have successive changeovers between two phases of ventilation (inspiration and expiration) by examining, in one phase of ventilation, a sensed signal for breathing activity for a threshold criterion for a changeover to the next phase of ventilation and changing the ventilator machine over from one phase of ventilation to the other when the threshold criterion is met and control the ventilator unit of the ventilator machine at the changeover to the new phase of ventilation in such a way that a pneumatic ventilation parameter (airway pressure, flow) is moved from an actual value to a target value for the new phase of ventilation which is set in advance.

The aim of the artificial maintaining of respiration with ventilator machines is to relieve the strain on a patient's respiratory muscles and to ensure that there is an adequate supply of oxygen and that carbon dioxide is eliminated to an adequate degree. This can be done by causing the ventilator machine to assume responsibility for the whole of the breathing activity or, in assisting techniques, for part of the breathing activity, existing breathing activity by the patient being assisted or boosted in these latter assisting techniques. For this purpose, the ventilator machines contain a ventilator unit to supply gas for breathing at a pressure which is preset by a control unit. Also present are sensors which sense, as a function of time, pneumatic breathing signals, such for example as the airway pressure, or the flow of the gas for breathing and its In view of the increase in chronic lung disease and the demand for an improved therapy, the non-invasive assistance of breathing with improved interaction between the patient and the ventilator unit is a crucial requirement which needs to be met by modern-day ventilator machines. A significant object to be achieved in this case is the establishing of temporal synchrony between the assistance provided by the machine and the patient's own breathing activity. In the past, what was often done was to sedate patients who were breathing spontaneously to allow the ventilation to be correctly set and to allow synchrony to be forced to exist between the patient and the ventilator machine. From the knowledge which now exists, this procedure is no longer acceptable because risks have to be run of the lungs being damaged by the ventilation.

For improved synchronisation between the patient's breathing activity and the action of the ventilator unit, it is important for the beginning of inspiration and the beginning of expiration to be reliably detected in the patient's breathing activity at an early point in time. Especially in the case of neonates and COPD patients, the detection of phases of breathing is often incorrect or late with conventional methods and results in increased breathing effort even going as far as exhaustion.

For artificial maintaining of respiration which is intended to take account of the patient's breathing activity in an improved way, it is known from DE 10 2007 062 214 B3 not only for pneumatic signals for breathing activity to be sensed but also for electromyographic signals to be picked up by electrodes on the thorax and for electromyographic signals for breathing activity (EMG signals) to be derived from these. These EMG signals are independent of the pneumatic signals for breathing activity and thus constitute an independent source of information which can be used to sense the beginning of inspiration and expiration. However it is not uncommon for the EMG signals to have interference and disruptions, such for example as the ECG signal from the heart, motion artefacts, or what is referred to as crosstalk (muscle activity which has nothing to do with the patient's respiratory system), superimposed on them.

For the last reason mentioned, EMG signals cannot serve as the sole basis for the detection of the beginning of inspiration and expiration and for the corresponding control of the ventilator machine.

Triggering of breaths on the basis of EMG signals is described in US 6,588,423 B1. In this case the raw EMG signal is pre-processed and, for triggering, a measure of the intensity of the EMG signal (the root mean square) is finally used, a threshold which is fixed, in relation to one breath, being used.

In practice however even the pre-processed EMG signal is generally more susceptible to interference or disruption than pneumatic signals (pressure or flow). A susceptibility of this kind to interference or disruption, or a volatility, makes it more difficult to change over between breaths or trigger them when trigger thresholds are used, because the incorrect triggering of too many breaths may occur (what is referred to as auto-triggering), or breaths may be triggered too late (what is referred to as delayed or missed triggering).

Although signals can be prevented from being affected by interference or disruptions by suitable filtering (e.g. by means of the formation of sliding means), for the purpose of making a changeover between phase of ventilation this has the serious disadvantage of causing an additional delay to the signals.

It is an object of the present invention to provide a ventilator machine which keeps the effects of wrongly initiated changeovers to the next phase of ventilation, i.e. from inspiration to expiration or from expiration to inspiration, as small as possible.

The present invention is as claimed in the claims.

In accordance with the invention, provision is made for, after the triggering of the changeover to the new phase of ventilation, the control of the ventilation parameter to the target value by the ventilator unit to be undertaken not in one stage but in a plurality of successive part-stages. After each part-stage, what is then the current signal for breathing activity is again examined for the threshold criterion responsible for the current changeover and, if this threshold criterion is then no longer met, a jump is automatically caused to the state of operation of the ventilator machine before the changeover which was currently triggered. In other words, a complete changeover to the next phase of ventilation is not made immediately and instead the changeover is carried out in part-stages and after each part-stage a check is made on the question of whether what was indicated by the threshold criterion which triggered the start of the changeover was an actual change of the phase of ventilation or interference or a disruption. If the threshold criterion is no longer met after the part-stage, this is an indication that the meeting of the threshold criterion at the time of the triggering in the first part-stage, and possibly other previous part-stages, was caused by interference or a disruption and that the ongoing waveform of the signal for breathing activity is no longer consistent with the assumption that a changeover to the next phase of ventilation should in fact be triggered. A wrongly triggered changeover can therefore be cancelled again after one or a few part-stages by virtue of the fact that the ventilator machine automatically jumps back to the last phase of ventilation before the changeover was triggered. In this way, a changeover which was wrongly triggered is broken off again at an early stage and a jump is made back to the correct phase of ventilation which existed before the wrongly triggered changeover, which should really still be continuing.

The effects of a wrongly triggered changeover are considerably reduced in this way, because it is not a complete inspiration or a complete expiration which elapses if a changeover is wrongly triggered by interference or a disruption. The effects of wrong changeovers are considerably reduced by the method according to the invention by this means, but what the method according to the invention also allows is for the threshold criteria to be preset in a more sensitive way, because a certain increase in wrongly met threshold criteria caused by interference or disruptions can be tolerated because of the considerably reduced effects of such wrongly triggered changeovers.

The concept of this "stage by stage" changeover, with a check each time on whether the signal for breathing activity is still consistent with the supposition of an actual transition to the next phase of ventilation, is preferably applied to signals for breathing activity which are not directly affected by the control carried out by the ventilator machine. The ventilator machine controls the pneumatic parameters of the ventilation process, and pneumatic signals for breathing activity such as airway pressure and flow are thus directly affected by the ventilator machine. Signals for breathing activity which are not of a pneumatic nature and which are thus not directly affected by the ventilator machine are for example electromyographic signals which are picked up by electrodes on the surface of the skin of the thorax. This is particularly the case because the electrical activity of the respiratory muscles does not react at once to a ventilator stroke. In the case of pneumatic signals (or ones derived therefrom), the time slot for a possible jump back to the previous phase of ventilation is limited due to the principle of operation because the ventilator unit reacts quickly when the threshold criterion is met and changes pneumatic signals for breathing activity quickly.

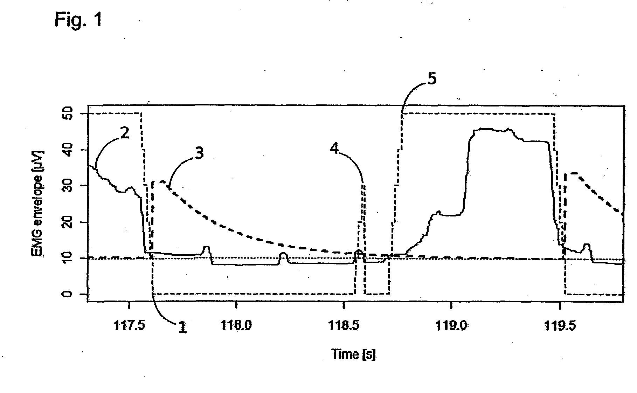

The present invention will now be described, by way of example, only, by reference to the accompanying Fig. 1 which is a graph which shows the waveform of the signal for breathing activity, the curve followed by the threshold criterion and the curve followed by the control of the ventilation parameter by the ventilator machine, all as functions of time.

What is first shown in Fig. 1 is a pre-processed EMG signal. Pre-processing of this kind of the raw EMG signal is performed in a known manner in such a way that the raw EMG signal is freed of interfering and disrupting signals (e.g. ECG, motion artefacts, mains hum), and finally, envelope curve detection is carried out. Envelope curve detection may for example be performed by "rectification" followed by low pass filtering, the "rectification" being performed by an operation which gives a value (e.g. squaring or pure absolute-value generation). After the low pass filtering, what is then obtained is the envelope curve, i.e. the curve which is an envelope enclosing the waveform of the raw signal. A preferred implementation of the envelope curve detection process is the formation of what is referred to as the RMS (root mean square) over the length of a sliding time slot. The concept of EMG amplitude estimation which can be described by the term "envelope curve detection" is described in detail in Merletti R, Parker P.A.: Electromyography, Physiology, Engineering and Noninvasive Applications, IEEE Press, Wiley Interscience, 2004, Chapter 6.4 et seq, i.e. page 139 ff.

This pre-processed EMG signal is identified as 2 in Fig. 1. The dashed curve identified as 3 is a threshold, a time-dependent dynamic threshold in the present case. The embodiment of the threshold is not basically of any significance to the present invention and the invention can also advantageously be applied with constant thresholds. However, what is also suitable in the present example for detecting the next inspiration is a dynamic threshold which first begins, at the start of an expiration, at a relatively high value. This relatively high value lasts for a preset period of time and the dynamic threshold is then reduced to a target value which should be reached at the time when the expiration is expected to end; the expected end point of the expiration may for example be estimated from the duration of the previous expiration phases. The preset period of time for which the threshold is held at a relatively high constant value after the beginning of the expiration is to be selected to be such that it is appreciably shorter than all the expected expiration phases, i.e. the likelihood of a new inspiration beginning as early as during this period of time should be vanishingly small; a period of time of this kind may for example be 200 ms. The target value for the threshold may for example be derived from the maximum and minimum values $u_{pneu}^{max}$, $u_{pneu}^{min}$, $u_{non-pneu}^{max}$ and $u_{non-pneu}^{min}$ of the signals in previous phases of ventilation.

Conversely, the threshold for detecting the next expiration begins, at the start of an inspiration, at a low value which lasts for a preset period of time and the threshold is then raised to a preset target value which should be reached at the time when the inspiration is expected to end. The expected end point of the inspiration may, in turn, be estimated from the duration of the previous inspiration phases and the target values may be derived from the maximum and minimum values $u_{pneu}^{max}$, $u_{pneu}^{min}$, $u_{non-pneu}^{max}$ and $u_{non-pneu}^{min}$ of the signals in previous phases of ventilation.

The consideration underlying dynamic thresholds of this kind is that it is precisely at the beginning of a phase of ventilation that there is a very small likelihood that a change to the next phase of ventilation might again take place as early as within the short period of time which is preset. It is therefore possible to use, at the beginning of a phase of ventilation, thresholds which are set in such a way that the possibility of an erroneous changeover can be virtually ruled out even if there is severe interference or disruption, whereas after the preset period of time the threshold is moved to a target value which is to be reached at the expected end of the phase of ventilation, thus enabling the beginning of the next phase of ventilation to be detected with great sensitivity. The expected value may correspond to the value which would be suitable for a sensitive constant threshold. This being the case, there is an assurance of sensitive triggering towards the end of the phase of ventilation but incorrect triggering as a result of interference or disruptions is suppressed at the beginning of the phase.

What is also shown in Fig. 1, by the thin dashed curve, is the curve followed by the ventilation parameter set by the ventilator machine, which in the present case is the curve followed by the flow preset by the ventilator machine.

The plot begins at the left-hand end with the residual part of an inspiration phase, meaning that flow (the thin dashed line) is set to a high constant value. The EMG signal 2 is already beginning to fall towards the end of this inspiration phase and at the end it drops steeply to a baseline value. At the end of this steep drop, the threshold criterion for the triggering of the changeover to expiration is met, whereupon the flow is brought down by the ventilator machine from the high constant value. However, this is not done in a single stage but in a plurality of part-stages, provision for a short delay being made after each part-stage, which means that flow can be seen to follow a staircase-like curve. After each part-stage of the staircase-like curve, or on each step thereof, the threshold criterion which triggered the changeover is again checked against the current signal for breathing activity. If the signal for breathing activity continues to meet the threshold criterion which triggered the changeover, the sequence of part-stages continues. This is the case with the changeover to expiration which is shown on the left of Fig. 1, because the signal for flow follows a curve which moves downwards in steps to the baseline value, at which it then remains for quite a long period.

In the expiration phase, from about 117.6 s on, the EMG signal remains largely low and all that can be seen are quite small humps in the signal (artefacts caused by the ECG signal), which however initially remain appreciably below the dynamic threshold 3. Towards the end of the expiration phase, an artefact of this kind again occurs, in the region which has been given reference numeral 4. However, the dynamic threshold is already relatively low at this time because the duration of the current expiration phase is already approaching the expected value for expiration phases. The artefact which occurs in the region of reference numeral 4 therefore causes the EMG signal to meet the threshold criterion for the changeover to the next phase of ventilation (inspiration). Consequently, the rise in the value of the ventilation parameter which has to take place after this is initiated by the ventilator machine. This rise in the flow once again takes place in part-stages. After each part-stage in the staircase-like curve followed by the rise in the flow, the threshold criterion which triggered this changeover is again checked against the signal for breathing activity which then exists, and the rise only continues if the threshold criterion is still met. In the region of the artefact 4, this is the case for three of the part-stages, i.e. three part-stages or steps of the rise are traversed. However, in the check, against the signal for breathing activity which then exists, which is then again made of the threshold criterion which triggered inspiration it is found that the threshold criterion is no longer met because the artefact in the region 4 lasts for only a relatively short time and after the third part-stage of the initiation of the inspiration has already decayed sufficiently far for the threshold criterion for changeover to inspiration no longer to be met. The changeover to inspiration is then broken off and the ventilator machine is reset to the state which existed before the initiation of inspiration in the region 4, i.e. to the expiration state. This expiration state then lasts a short further time.

After this, the EMG signal begins to show a sustained rise in activity. This first results in the threshold criterion for the changeover to inspiration again being met. The setting of the ventilation parameter of flow to the inspiration state in a plurality of part-stages (there are five part-stages in the example shown) then begins. Because the EMG undergoes a protracted raise at this time, the beginning of a true inspiration, the step-like rise in flow is continued without a jump back and the changeover to inspiration is maintained for a protracted period. After this, the process continues in the manner described above with inspiration until the changeover to the next phase of ventilation takes place on the right of Fig. 1.

The constant phase after each part-stage may for example be about 50 ms. During this time the triggering threshold criterion is checked and, if it no longer meets the condition for assuming a transition to the next phase of ventilation, a jump is made back to the state of ventilation for the ventilator machine which applied before the changeover was initiated. The reason for the threshold criterion no longer being met may equally well be a change of the signal for breathing activity and a change in a dynamic threshold which occurred after the first part-stage was triggered; the latter case however is not so important because dynamic thresholds change very slowly in the periods covered by the part-stages of the changeover, particularly towards the end of a phase of ventilation. If however the threshold criterion is still met after a part-stage, the next part-stage takes place to the next step. Once the last step is reached, this is equivalent to a complete change to the next phase of ventilation.

The number of part-stages is preferably two to ten. The time for the changeover of airway pressure or flow to the preset target value is typically selected to be in a range from 100 to 300 ms, preferably as approximately 200 ms. This corresponds to a duration for an individual part-stage of approximately 20 ms to 100 ms.

The part-stages may be uniform in the sense of the width of the stage in the change in the ventilation parameter being the same for all the part-stages. It is preferable however for the width of the first part-stage to be smaller than that of the at least one part-stage which follows it. From the first to the last part-stage, the width of the stages may also increase from stage to stage or after a plurality of part-stages. A procedure where the width of the stages increases has the advantage that first it is only quite small widths of stage which are selected for the first part-stage or part-stages, because the likelihood of the triggering of the changeover being caused by interference or a disruption, and of a jump back to the last state of ventilation being necessary, is greatest shortly after the beginning of the changeover. Once part-stages have already taken place it becomes increasingly improbable that the changeover which has already taken place was caused by interference or a disruption because the threshold criterion will then already have proved to be met over a plurality of past part-stages.

Provision may be made for the change in the desired value in each part-stage to be limited to a selectable maximum value of the rate of change. This is in fact also what is preferred, which means that the sequence of part-stages is not a true staircase function having vertical or almost vertical rises and declines but there is a continuous rise in each part-stage. If the maximum value of the rate of change is suitably selected, the result may be a continuous linear rise in each part-stage and the sequence of part-stages will thus, as a whole, then show a ramp-like rise from the actual value to the desired value. The jump back to the state in the previous phase of ventilation after a changeover which had begun is broken off should of course take place as quickly as possible and because of this no provision is made for limiting the rate of change for this.

The limiting of the rate of change is preferably applied to the desired value curve for the ventilation parameter but may for example also be implemented in the form of a short-term change in the characteristics of the ventilator machine as a controller. The limitation $v_{max}$ on the rate of change is preferably matched to the width T and height $h_i$ of the $i^{th}$ part-stage, e.g. by means of the formula $v_{max} = h_i/T$.

As an alternative, rather than the rate of change of the desired value curve being limited, the desired value curve may be subjected to low pass filtering which results in the staircase signal being smoothed. The limit frequency f of the low-pass filter is preferably matched to the width T of the part-stages, e.g. by means of the formula $f = 1/(2\pi T)$.

## Claims

1. A ventilator machine having:

   a source of gas for breathing;
   a ventilator unit arranged to feed gas for breathing through connecting lines;
   a control unit arranged to control the ventilator unit;
   sensors arranged to pick up at least one pneumatic signal for breathing activity which are connected to the control unit; the control unit being arranged to:

   control the ventilator unit of the ventilator machine to have successive changeovers between two phases of ventilation, the two phases being inspiration and expiration, by examining, in one phase of ventilation, a sensed signal for breathing activity for a threshold criterion for a changeover to the next phase of ventilation and changing the ventilator machine over from one phase of ventilation to the other when the threshold criterion is met; and
   control the ventilator unit of the ventilator machine at the changeover to the new phase of ventilation in such a way that a pneumatic ventilation parameter , selected from airway pressure and flow, is moved from an actual value to a target value for the new phase of ventilation which is set in advance; the ventilator machine being **characterised in that** the control unit is also arranged to:
   divide the change in the breathing parameter from the actual value to the target value into a plurality of part-stages at the changeover of the phase of ventilation and, after each part-stage, to again examine the current signal for breathing activity for the threshold criterion responsible for the triggering of the current changeover and, if this threshold criterion is no longer met, to jump back automatically to the state of operation of the ventilator machine before the last changeover, or otherwise to continue to the next part-stage.

2. The ventilator machine according to claim 1, in which the widths of the part-stages from the desired value to the actual value are so selected that the width of the first part-stage is smaller than that of the at least one subsequent part-stage.

3. The ventilator machine according to claim 2, in which the widths of the part-stages from the desired value to the actual value are so selected that the width of the part-stages increases from the first part-stage to the last from part-stage to part-stage or in a plurality of steps.

4. The ventilator machine according to one of the preceding claims, in which the rate of change of the ventilation parameter in each part-stage is limited to a selectable maximum value.

5. The ventilator machine according to one of the preceding claims, in which the desired value curve provided for controlling the ventilation parameter from the actual value to the desired value is subjected to low pass filtering.

6. The ventilator machine according to claim 5, in which the limit frequency f of the low pass filter is matched to the width T of the part-stages, preferably by means of the formula $f = 1/(2\pi T)$.

**Patentansprüche**

1. Beatmungsgerät, welches Folgendes aufweist:

> eine Atemgasquelle;
> eine Ventilatoreinheit, die zum Zuführen von Atemgas durch Verbindungsleitungen geeignet ist;
> eine Steuereinheit, die zum Steuern der Ventilatoreinheit geeignet ist;
> Sensoren, die zum Aufnehmen mindestens eines pneumatischen Signals für die Atmungsaktivität geeignet sind, welche mit der Steuereinheit verbunden sind;
> wobei die Steuereinheit zu Folgendem geeignet ist:

> Steuern der Ventilatoreinheit des Beatmungsgerätes, derart, dass sie aufeinanderfolgende Umschaltungen zwischen zwei Phasen der Beatmung aufweist, wobei die beiden Phasen Inspiration und Exspiration sind, durch Prüfung, in einer Phase der Beatmung, eines erkannten Signals für die Atmungsaktivität auf ein Schwellenkriterium für eine Umschaltung in die nächste Phase der Beatmung und Umschaltung des Beatmungsgerätes von einer Phase der Beatmung in die andere, wenn das Schwellenkriterium erfüllt ist; und
> Steuern der Ventilatoreinheit des Beatmungsgerätes bei der Umschaltung in die neue Phase der Beatmung, derart, dass ein pneumatischer Beatmungsparameter, ausgewählt aus Atemwegsdruck und Durchflussmenge, für die neue Phase der Beatmung von einem Istwert zu einem vorab eingestellten Zielwert bewegt wird;
> wobei das Beatmungsgerät **dadurch gekennzeichnet ist, dass** die Steuereinheit auch zu Folgendem geeignet ist:

> Aufteilen der Änderung im Atmungsparameter von dem Istwert zu dem Zielwert in mehrere Teilstufen bei der Umschaltung der Phase der Beatmung und, nach jeder Teilstufe, erneute Prüfung des aktuellen Signals für die Atmungsaktivität auf das Schwellenkriterium, das für das Auslösen der aktuellen Umschaltung verantwortlich ist, und, falls dieses Schwellenkriterium nicht mehr erfüllt ist, automatisches Springen zurück in den Betriebszustand des Beatmungsgerätes vor der letzten Umschaltung, oder stattdessen Fortfahren mit der nächsten Teilstufe.

2. Beatmungsgerät nach Anspruch 1, wobei die Breiten der Teilstufen von dem Sollwert zu dem Istwert derart ausgewählt sind, dass die Breite der ersten Teilstufe geringer als die der mindestens einen nachfolgenden Teilstufe ist.

3. Beatmungsgerät nach Anspruch 2, wobei die Breiten der Teilstufen von dem Sollwert zu dem Istwert derart ausgewählt sind, dass sich die Breite der Teilstufen von der ersten Teilstufe zu der letzten von Teilstufe zu Teilstufe oder in mehreren Schritten erhöht.

4. Beatmungsgerät nach einem der vorhergehenden Ansprüche, wobei die Änderungsgeschwindigkeit des Beatmungsparameters in jeder Teilstufe auf einen auswählbaren Maximalwert begrenzt wird.

**5.** Beatmungsgerät nach einem der vorhergehenden Ansprüche, wobei der zur Führung des Beatmungsparameters von dem Istwert zu dem Sollwert vorgesehene Sollwertverlauf einer Tiefpassfilterung unterzogen wird.

**6.** Beatmungsgerät nach Anspruch 5, wobei die Grenzfrequenz f des Tiefpassfilters an die Breite T der Teilstufen angepasst ist, vorzugsweise durch die Formel f = 1/(2π T).

**Revendications**

**1.** Respirateur comprenant :

une source de gaz pour la respiration ;
une unité de ventilation agencée de manière à introduire du gaz pour la respiration par l'intermédiaire de conduites de connexion ;
une unité de commande agencée de manière à commander l'unité de ventilation ;
des capteurs agencés de manière à capter au moins un signal pneumatique d'activité de respiration qui sont connectés à l'unité de commande ; l'unité de commande étant agencée de façon à :

commander l'unité de ventilation du respirateur de manière qu'il y ait des passages successifs entre deux phases de ventilation, les deux phases étant une inspiration et une expiration, par examen, dans une première phase de ventilation, d'un signal capté d'activité de respiration pour un critère de seuil de passage à la phase suivante de ventilation, et changement du respirateur d'une première phase de ventilation à l'autre quand le critère de seuil est atteint ; et
commander l'unité de ventilation du respirateur lors du passage à une nouvelle phase de ventilation de façon qu'un paramètre de ventilation pneumatique, choisi parmi la pression des voies aériennes et le débit, soit déplacé d'une valeur réelle à une valeur cible pour la nouvelle phase de ventilation qui est établie à l'avance ; le respirateur étant **caractérisé en ce que** l'unité de commande est également agencée de façon à :

diviser le changement du paramètre de respiration de la valeur réelle à la valeur cible en une pluralité de stades partiels lors du changement de phase de ventilation et, après chaque stade partiel, examiner de nouveau le signal en cours d'activité de respiration pour le critère de seuil responsable du déclenchement du passage en cours, et si ce critère de seuil n'est plus satisfait, revenir automatiquement à l'état de fonctionnement du respirateur avant le dernier passage, ou sinon poursuivre avec le stade partiel suivant.

**2.** Respirateur selon la revendication 1, dans lequel les largeurs des stades partiels entre la valeur souhaitée et la valeur réelle sont sélectionnées de façon que la largeur du premier stade partiel soit inférieure à celle de l'au moins un stade partiel subséquent.

**3.** Respirateur selon la revendication 2, dans lequel les largeurs des stades partiels entre la valeur souhaitée et la valeur réelle sont sélectionnées de façon que la largeur des stades partiels augmente du premier stade partiel au dernier stade partiel d'un stade partiel à un autre stade partiel ou en une pluralité d'étapes.

**4.** Respirateur selon l'une des revendications précédentes, dans lequel le taux de changement du paramètre de ventilation dans chaque stade partiel est limité à une valeur maximale sélectionnable.

**5.** Respirateur selon l'une des revendications précédentes, dans lequel la courbe de valeur souhaitée fournie pour la commande du paramètre de ventilation entre la valeur réelle et la valeur souhaitée est soumise à une filtration passe-bas.

**6.** Respirateur selon la revendication 5, dans lequel la fréquence limite f du filtre passe-bas correspond à la largeur T des stades partiels, de préférence au moyen de la formule f = 1/(2π T).

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102007062214 B3

- US 6588423 B1

**Non-patent literature cited in the description**

- **MERLETTI R ; PARKER P.A.** Electromyography, Physiology, Engineering and Noninvasive Applications. IEEE Press, 2004, 139 ff